# EUROPEAN PATENT APPLICATION

(11) **EP 3 771 909 A1**
(43) Date of publication of application: **03.02.2021**
(21) Application number: 19189239.7
(22) Date of filing: 30.07.2019
(51) Int. Cl.: G01N 33/574, A61K 39/00

(54) **ANALYTICAL METHOD AND IMMUNOLOGICAL TREATMENT FOR BLADDER CANCER**

(71) Applicant: Medizinische Hochschule Hannover, 30625 Hannover (DE)
(72) Inventor: Büttner, Falk, 30625 Hannover (DE); Rossdam, Charlotte, 30625 Hannover (DE); Gerardy-Schahn, Rita, 30625 Hannover (DE); Oberbeck, Astrid, 30625 Hannover (DE); Tezval, Hossein, 30625 Hannover (DE)
(74) Representative: Taruttis, Stefan Georg

(57) **Abstract**

The invention provides an analytical method for detecting bladder cancer by analysing a urine sample for presence of a glycan tumour antigen, the presence of which has been identified to indicate bladder cancer. The analytical method has the advantage that the tumour antigen can be analysed in a urine sample and has a high accuracy for indicating presence of bladder cancer, e.g. the method has a low rate of false positive results.

## Description

The present invention relates to an analytical method for detecting bladder cancer by analysing a urine sample for presence of at least one specific tumour antigen. Further, the invention relates to compounds for use in the treatment of bladder cancer, e.g. for use in a method of treatment of bladder cancer, wherein the compounds are specific for the tumour antigen. In this embodiment, the compound can e.g. be an antibody specific for the tumour antigen.

The tumour antigen provided by the invention has the advantage that its detection in a urine sample is specific for the presence of bladder cancer. Optionally, the tumour antigen is analysed in combination with a further tumour antigen which has been found to also indicate presence of bladder cancer, and/or is analysed in combination with one or two further analytes for which elevated levels in urine have been found to indicate absence of bladder cancer.

The invention also relates to an immunological treatment of bladder cancer by providing a peptide binding to the tumour antigen for use in the treatment of bladder cancer. The peptide binding to the tumour antigen can e.g. be an antibody or a lectin or be a part of a chimeric antigen receptor (CAR) for expression in an immune cell. The peptide binding to the tumour antigen may be an immunoglobulin, e.g. IgG, IgM, IgA, or IgG, or an antigen binding fragment thereof, or an scFv.

Further, invention relates to compounds for use in the treatment of bladder cancer, wherein the compounds contain or consist of the at least one specific tumour antigen, e.g. the at least one tumour antigen for use in the treatment of bladder cancer.

### State of the art

Deb and Kumar, Journal of Cancer Research and Treatment, 34-38 (2018) recite several proteinaceous molecules and micro RNAs that had been identified as possible indicators of bladder cancer found in urine, and conclude that tumour heterogeneity would not allow for use of a single biomarker.

Azevedo et al., Oncotarget 91734-91764 (2017) review glycoproteins, glycolipids and proteoglycans that have been found in bladder cancer.

Ohyama, Int J Clin Oncol 308-313 (2008) reviews carbohydrate synthesis by normal and tumour cells and the role of glycoproteins, proteoglycans and glycosphingolipids in cellular interactions, quoting that the blood group carbohydrate antigen Lewis X could be shown to be present in up to 90% of bladder cancer tumours, e.g. on exfoliated cells of urine samples, by a monoclonal anti-Lewis X antibody.

WO 2007/023191 A2 describes 836 peptides identifiable by retention times in capillary electrophoresis as markers for bladder cancer.

Rossdam et al., Anal. Chem. 2019, 91, 6413-6418, describe an analytical method for the analysis of glycosphingolipid glycosylation by multiplexed capillary gel electrophoresis coupled to laser-induced fluorescence detection (xCGE-LIF) for the identification of cell surface markers of human induced pluripotent stem cells and derived cardiomyocytes.

### Object of the invention

It is an object of the invention to provide a bladder tumour-specific antigen that is suitable for analysis in a urine sample and indicates presence, respectively absence, of bladder cancer. A further object is to provide a new therapeutic target specific for bladder cancer, and to provide a compound for use in the treatment of bladder cancer.

### Description of the invention

The invention achieves the object by the features of the claims, especially by providing an analytical method for detecting bladder cancer by analysing a urine sample for presence of a tumour antigen, the presence of which has been identified to indicate bladder cancer. The analytical method has the advantage that the tumour antigen can be analysed in a urine sample and has a high accuracy for indicating presence of bladder cancer, e.g. the method has a low rate of false positive results. According to the invention, the tumour antigen is the glycan consisting of lacto-N-neo-tetraose (Galβ1-4GlcNAcβ1-3Galβ1-4Glc, nLc4), which originates from the glycosphingolipid neolactotetraosyl ceramide and which was found to be highly elevated in urine samples of patients with bladder cancer compared to urine samples of persons free from bladder cancer. Optionally, nLc4 is analysed in combination with lactose (Galβ1-4Glc) as an additional tumour antigen, which originates from the glycosphingo lipid lactosyl ceramide and which has been identified to be augmented in urine samples of patients with bladder cancer as well. Further additionally, the glycans globotetraose (GalNAcβ1-3Galα1-4Galβ1-4Glc, Gb4) and globotriaose (Galα1-4Galβ1-4Glc, Gb3), originating from the glycosphingolipids globotetraosyl ceramide and globotriaosyl ceramide, respective, are provided as a negative indicators for bladder cancer. These latter glycans have been found at higher concentrations in urine of healthy controls compared to patients with bladder cancer. Accordingly, high concentrations of nLc4 and of lactose, optionally in combination with low concentrations of Gb4 and of Gb3 indicate presence of bladder cancer, whereas low concentrations of nLc4 and lactose in combination with high concentrations of Gb4 and Gb3 indicate absence of bladder cancer. Both, the bladder cancer positive indicators nLc4 and lactose as well as the bladder cancer negative indicators Gb4 and Gb3 originate from glycosphingolipids. In the analytical method, the presence of the glycan consisting of lacto-N-neo-tetraose (nLc4), which preferably originates from neolactotetraosyl ceramide, was found to be indicative of bladder cancer. In contrast, elongated glycosphingolipid glycans that are derived from nLc4, e.g. glycans having a longer chain of sugar moieties than nLc4, e.g. fucosyl-lacto-N-neo-tetraose or sialyl-lacto-N-neo-tetraose having an additional fucose or sialic acid attached to the structure of nLc4, respectively, have been found not to be indicative of bladder cancer. Accordingly, glycans having additional sugar moieties attached to the structure of lacto-N-neo-tetraose are preferably excluded from the present invention, e.g. excluded from the antigens to be analysed, and excluded from the antigen for which a binding peptide has specificity, e.g. against which an antibody or a CAR is directed.

In the invention, the glycan consisting of nLc4, optionally additionally the glycan consisting of lactose, optionally additionally the glycan consisting of Gb4 and/or Gb3, can be determined as the free glycan consisting of nLc4, of lactose, respectively of Gb4 and/or Gb3, or, alternatively, these glycans can be determined as their respective glycosphingolipids, i.e. as neolactotetraosyl ceramide, lactosyl ceramide, globotetraosyl ceramide, and/or globotriaosyl ceramide, respectively.

In the analytical method, the free glycans nLc4, lactose, Gb4, and/or Gb3 can be determined after hydrolysis of the respective glycosphingolipids, i.e. after hydrolysis of neolactotetraosyl ceramide, of lactosyl ceramide, of globotetraosyl ceramide, and/or globotriaosyl ceramide e.g. by chemical or enzymatic hydrolysis with ceramide glycanases or endoglycoceramidases directly in the urine sample or, preferably, by hydrolysis of the lipid fraction generated, e.g. isolated, from the urine sample. Alternatively the respective glycosphingolipids neolactotetraosyl ceramide, lactosyl ceramide, globotetraosyl ceramide, and/or globotriaosyl ceramide can be determined directly in the urine sample or, preferably, in the lipid fraction isolated from the urine sample.

A lipid fraction can be generated from the urine sample e.g. by adsorption to a lipophilic adsorbent, preferably by extraction of the urine sample with organic solvents, and separating the lipophilic phase from the aqueous phase, optionally with removing a lipophilic adsorbent, respectively removing the organic solvents.

Optionally, in the analytical method, a membrane fraction is prepared from the urine sample, from which a lipid fraction can be prepared, e.g. by adsorption of the membrane fraction to a lipophilic adsorbent, preferably by extraction of the membrane fraction with organic solvents, preferably with subsequently separating the lipophilic adsorbent, respectively removing the organic solvents. The membrane fraction can be prepared by concentrating and separating the cells and/or membrane vesicles from the urine sample, e.g. by centrifugation or filtration, and removing the cell-free and/or the membrane vesicle-free fraction. Optionally, a fraction containing essentially only membrane vesicles is isolated from the urine sample, which fraction preferably is cell-free, e.g. by firstly removing cells, optionally also cell-debris, from the urine sample, e.g. by centrifugation or by filtration, and secondly concentrating membrane vesicles from the remaining liquid phase, e.g. by ultracentrifugation or by membrane filtration.

Optionally, a detergent may be added to the urine sample or a membrane fraction thereof, or a lipid fraction thereof, in order to solubilize neolactotetraosyl ceramide, lactosyl ceramide, globotetraosyl ceramide, and/or globotriaosyl ceramide, optionally with subjecting the mixture with the detergent to agitation and/or to ultrasound.

An elevated molar concentration of the glycan consisting of nLc4, respectively of its ceramide neolactotetraosyl ceramide, optionally additionally an elevated molar concentration of lactose, respectively of lactosyl ceramide, optionally additionally a lower molar concentration of Gb4, respectively of globotetraosyl ceramide, and/or optionally additionally a lower molar concentration of Gb3, respectively of globotriaosyl ceramide indicates presence of bladder cancer. The elevated or lower molar concentration can be determined in comparison to the concentration, preferably to the mean concentration, determined from urine samples from healthy persons. For example, an elevated molar concentration of nLc4 is higher by a factor of at least 1.5, a factor of at least 2, a factor of at least 3, a factor of at least 4, a factor of at least 5, a factor of at least 6 or a factor of at least 7 or of at least 10 or at least 15 or at least 20 than the mean concentration of nLc4 determined from the urine of healthy persons.

Advantageously, it was found that the molar concentrations of nLc4, of lactose, of Gb4, and of Gb3 in a healthy person are essentially independent from the age of the person. Further it was found that the molar concentrations of nLc4, of lactose, of Gb4, and of Gb3 in urine samples originating from patients diagnosed with bladder cancer were essentially independent from the ages of the patients.

Preferably, the molar concentration is determined as the relative molar concentration, i.e. as the proportion of the total molar concentration of glycans analysed, e.g. the total molar concentration of glycosphingolipids, or of free glycans of glycosphingolipids. Optionally, the molar concentration is determined in relation to the molar concentration of a protein and/or metabolite, e.g. albumin and/or creatinine, respectively, which protein or metabolite is present in urine. Preferably, the protein or metabolite has a concentration in urine which is not altered in patients with bladder cancer compared to persons without bladder cancer.

An elevated molar concentration of nLc4, or of neolactotetraosyl ceramide, indicating presence of bladder cancer can be determined as at least 1%, at least 2%, at least 3%, at least 4%, at least 5%, at least 6%, at least 7%, at least 8%, at least 9%, e.g. at least 10%, of the total signal intensities measured for glycans, respectively for glycosphingolipids, in a urine sample, preferably in a membrane vesicle fraction of a urine sample.

The molar concentration of nLc4, optionally additionally of lactose, optionally additionally of Gb4, and/or Gb3 and/or of their respective glycosphingolipids can be determined by capillary gel electrophoresis coupled to multiplexed laser-induced fluorescence detection (xCGE-LIF), by chromatography, e.g. coupled to a mass spectrometer or based on fluorescence detection, or by mass spectrometry. In the analysis, especially for capillary gel electrophoresis, it is preferred that the sample is reacted with a marker that labels each glycan in a pre-determined quantitative manner, e.g. that labels only the reducing end of the glycan by a pre-determined number of marker molecules, e.g. that labels the reducing end of each glycan by 1 marker molecule. An exemplary marker is the fluorescent dye 8-aminopyrene-1,3,6-trisulfonic acid (APTS), which labels each reducing end of a glycan with 1 dye molecule. Glycan analysis by xCGE-LIF depends on determination of glycan-specific migration time units (MTUs). Glycan-specific migration times can be pre-determined once by analysis of the glycans, e.g. available as glycan standards, including nLc4, lactose, Gb4 and Gb3. Further preferred, in xCGE-LIF, an internal standard labelled with a different dye is added to the glycans prior to analysis by xCGE-LIF, and the MTUs of the internal standard are used for standardizing the MTUs of the glycans.

The molar concentration of nLc4, optionally additionally the molar concentration of lactose, optionally additionally the molar concentration of Gb4, and/or the molar concentration of Gb3, as well as the respective glycosphingolipids from which these glycans originate, can be alternatively determined immunologically, using a peptide having binding specificity to nLc4, optionally additionally using a peptide having binding specificity to lactose, optionally additionally using a peptide having binding specificity to Gb4 and/or a peptide having binding specificity to Gb3. Preferably, the peptide having binding specificity to one of nLc4, lactose,Gb4, and Gb3 is an antibody or a lectin.

Generally, processes for generating an antibody having specificity for a specific antigen, herein the glycan consisting of Lc4, optionally additionally for the antigen consisting of lactose, optionally additionally for the glycan consisting of Gb4 and/or consisting of Gb3 are known, e.g. the hybridoma technology comprising fusing a tumour cell line with spleen cells of an animal experimentally vaccinated with the antigen, with subsequent screening of antibody producing hybridoma cells for secretion of a specific antibody. Further, the antigen binding section of an antibody can be generated by phage display methods and panning.

In a further embodiment, the invention also relates to nLc4, nLc4-ceramide or a nLc4-protein conjugate or a nLc4-derivative for use in the treatment of bladder cancer.

The invention is now described by way of examples and with reference to the figures, which show in
- Fig. 1 electropherograms of urine samples of healthy persons, and
- Fig. 2 electropherograms of urine samples of bladder cancer patients.
- Fig. 3 a dot plot of relative signal intensities for a glycan consisting of nLc4 determined from urine samples of healthy persons and in urine samples of bladder cancer patients,
- Fig. 4 a dot plot of relative signal intensities for a glycan consisting of lactose determined from urine samples of healthy persons and in urine samples of bladder cancer patients,
- Fig. 5 a dot plot of relative signal intensities for a glycan consisting of Gb4 determined from urine samples of healthy persons and in urine samples of bladder cancer patients,
- Fig. 6 a dot plot of relative signal intensities for the glycan Gb3 determined from urine samples of healthy persons and in urine samples of bladder cancer patients,
- Fig. 7 a comparison of relative signal intensities determined for glycans from urine samples of healthy persons (left bars) and in urine samples of bladder cancer patients (right bars) for the MTUs indicated below and between the bars,

### Example 1: Detection of bladder cancer by xCGE-LIF analysis of glycans in urine samples

As an example for an analytical method, xCGE-LIF was used for the analysis of glycans that were obtained by hydrolysis of glycosphingolipids by ceramide glycanase of the membrane-vesicle fraction obtained from the cell-free liquid fraction of urine samples. In detail, urine samples, e.g. 40 mL, were centrifuged at 200 x g for 20 min at 4°C, and the supernatant was centrifuged at 2000 x g for 20 min at 4°C to prepare a liquid cell-free fraction. This cell-free fraction was ultra-centrifuged in a Beckman-Coulter Optima L-100K centrifuge at 12500 rpm (16039 x g) for 20 min at 4°C and filtrated through a 0.22 µm membrane filter for removing residual cell debris. The liquid cell-free urine fraction was then ultra-centrifuged in a Beckman-Coulter Optima L-100K centrifuge using a Type 70 Ti rotor at 34200 rpm (120066 x g) for 70 min at 4°C to pellet the membrane-vesicle fraction. The supernatant was removed, and the pellet containing the membrane vesicles was washed by resuspending in 5 mL PBS (phosphate buffered saline) and again ultra-centrifuged using a Type 70 Ti rotor at 34200 rpm (120066 x g) for 70 min at 4°C to pellet the membrane vesicle fraction again. The pellet was resuspended in 1 mL 1:2 v/v chloroform/methanol, preferably by firstly adding the methanol portion and then adding the chloroform portion. Solubilisation was preferably supported by exposition to ultrasound. The solubilized chloroform/methanol mixture was centrifuged at 1620 x g for 5 min, and the supernatant was collected. The extraction of glycosphingolipids from the pellet was repeated twice using chloroform/methanol (2:1 (v/v)) and chloroform/methanol (1:1 (v/v)), respectively and the supernatants of the different extraction steps were pooled. The pooled supernatants were evaporated to dryness be removing the solvent, e.g. by a nitrogen stream. The dried glycolipids were dissolved in 1 mL methanol plus 2 mL water and then desalted applying on a Chromabond® C₁₈ ec polypropylene column that was pre-equilibrated with a sequence of chloroform, then 1:1 v/v chloroform/methanol, then methanol, then water. After applying the solubilized glycolipids to the pre-equilibrated column, the column was washed 3 x with water, then the glycolipids were eluted by methanol. The eluted glycolipids were again evaporated to dryness under nitrogen gas and stored at -20 °C until further use.

The eluted glycolipids were hydrolysed by ceramide glycanase derived from *Hirudo medicinalis* (GCase, obtained from Ludger, Oxfordshire, GB) in LudgerZyme ceramide glycanase reaction buffer for 24h at 37°C, or with endoglycoceramidase I (EGCase I, obtained from NewEngland Biolabs, Ipswich, USA) from *Rhodococcus triatomea* in the reaction buffer provided at 37°C for 16h. These enzymes cleave the β-glycosidic bond of glycosphingolipids and release the free glycan with a reducing end. For xCGE-LIF, the free glycans were fluorescence-labelled by APTS, obtained from Merck, Darmstadt, Germany.

The glycans were mixed with 2 µL 20 mM APTS in 3.5 M citric acid, 2 µL 2-picoline borane complex (2 M in DMSO, obtained from Merck) and 2 µl water and incubated for 16.5 h at 37°C in the dark. The reaction was stopped by adding 100 µL acetonitrile/water (80:20). Excess reagents were removed by hydrophilic liquid interaction chromatography applying Biogel P-10 gel (BioRad, Hercules, USA) in water/ethanol/acetonitrile (70:20:10) applied to the GHP membrane of a Pall Nanosep MF Centrifugal device (0.45 µm pore size, obtained from Merck). For pre-equilibration of the gel, solvent was removed by centrifugation at 54 x g for 1 min, and the gel was washed 3 x with water and then equilibrated with acetonitrile. Samples were applied to the suspended gel in 100 µL acetonitrile/water (80:20) with agitation for 5 min, liquid was removed by centrifugation at 54 x g for 1 min, discarding flow through, washing the gel 5 x with acetonitrile/water (80:20) containing 100 mM triethylamine (pH 8.5), then 3 x acetonitrile/water (80:20), wherein in each washing step the gel was shaken for 1 min at 450 rpm and centrifuged at 54 x g for 1 min. Elution of labelled glycans was with done for three times with water by centrifugation of the gel upon incubation for 5 min at 450 rpm by shaking, respectively. The pooled eluates were then concentrated (SpeedVac) by evaporating the water in the dark and optionally stored at -20°C.

For xGCE-LIF, the resulting APTS-labelled glycans were suspended in water, e.g. 30 µL, LC-MS grade (Merck), and 3 µl of glycans were mixed with 1 µL GeneScan 500 LIZ dye size standard (DNA fragments of 20 to 500 bp for use in calibration of MTU, 1:50 dilution in Hi-Di Formamide (obtained from Applied Biosystems, Thermo Fisher Scientific, Foster City, CA)) and added up to 10 µL with Hi-Di formamide in a MicoAmp Optical 96-well reaction plate (obtained from Applied Biosystems, Thermo Fisher Scientific, Foster City, CA, USA). The mixture of ATPS-labelled glycans and the LIZ-labelled size standard was separated by multiplexed capillary gel electrophoresis (xCGE) on an ABI PRISM 3100-Avant Genetic Analyzer, remodeled by Axel Künzler, advanced biolab service GmbH, Munich, Germany. The analyser was equipped with an array of 4 capillaries of 50 cm length that were filled with POP-7 polymer (obtainable from Applied Biosystems, Thermo Fisher Scientific). Running buffer was obtained from Applied Biosystems, Thermo Fisher Scientific. Samples were injected for 15 s at 1.6 kV, and after a data delay of 50 s, detection was for 30 min at 12 kV at 60°C using laser induced fluorescence detection and for analysis, the GeneMapper software v. 3.7 was used. For each measurement, an electropherogram was obtained displaying peak values for the size standard in one channel and for APTS-labelled glycans in another channel at specific migration time units (MTU) and with specific intensities (relative fluorescence units, RFU). A peak amplitude threshold of at least 10 RFU was set in the GeneMapper software for defining peaks.

For identification, known glycans were labelled with APTS and their MTUs were measured. Known glycans were used directly or after exoglycosidase digest using known enzymes. The following glycans were obtained and used as standards:

| product name | structure | supplier | name of glycan |
|---|---|---|---|
| Glyko® Lacto-N-Fucopentaose III (LNFPIII) | Galβ1-4(Fucα1-3)GlcNAcβ1-3Gaβ1-4Glc | Prozyme | Lewis^{x} pentaose |
| Glyko® Lacto-N-Fucopentaose I (LNFPI) | Fucα1-2Galβ1-3GlcNAcβ1-3Galβ1-4Glc | Prozyme | fucosyl Lc4 / SSE45 |
| Blood group H antigen pentaose type 2/ Lacto-N-neofucopentaose I (LnNFPI) | Fucα1-2Galβ1-4GlcNAcβ1-3Galβ1-4Glc | Elicityl-OligoTech® | fucosyl nLc4 |
| LS-Tetrasacchande a (LSTa) | Neu5Acα2-3Galβ1-3GlcNAβ1-3Galβ1-4Glc | Elicityt-OligoTech® | sialyl Lc4 |
| SSEA-4 hexaose | Neu5Acα2-3Galβ1-3GalNAcβ1-3Galα1-4Galβ-4Glc | Elicityl-OligoTech® | sialyl Gb5 / SSEA4 |
| Globo H | Fucα1-2Galβ1-3GalNAcβ1-3Galα1-4Galβ1-4Glc | Elicityl-OligoTech® | fucosyl Gb5 / globoH |
| Globotriaose (Gb3) | Galα1-4Galβ1-4Glc | Elicityl-OligoTech® | Gb3 |
| Lacto-N-neotetraose (LNnT) | Galβ1-4GlcNAcβ1-3galβ1-4Glc | Elicityl-OligoTech® | nLc4 |
| Globopentaose (Gb5) | Galol-3GalNAcαl-3Galαl-4Galβl-4GIc | Elicityt-OligoTech® | Gb5 / SSEA3 |
| Blood group A antigen hexaose type 1 | GalNAcα1-3(Fucα1-2)Galβ1-3GlcNAcβ1-3Galβ1-4Glc | Elicityt-OligoTech® | A type 1 hexa |

Further, the following glycosphingolipids (GSL) were obtained and, 50 ng each, were hydrolysed and APTS-labelled as described above and used as standards:

| product name | structure | supplier | trivial name |
|---|---|---|---|
| Ganglioside GM3 | Neu5Acα2-3Galβ1-4Glcβ1Cer | Avanti® | GM3 |
| Disialoganglioside GD₁ₐ | Neu5Acα2-3Galβ1-3GalNAcβ1-4(Neu5Acα2-3)Galβ1-4Glcβ1Cer | Merck | GD1a |
| Ganglioside GD₃ | Neu5Acα2-8Neu5Acα2-3Galβ1-4Glcβ1Cer | Merck | GD3 |
| Ganglioside GD_{1b} | Galβ1-3GalNAcβ1-4(Neu5Acα2-8Neu5Acα2-3)Galβ1-4Glcβ1Cer | Merck | GD1b |
| Ganglioside GD₂ | GalNAcβ1-4(Neu5Acα2-8Neu5Acα2-3)Galβ1-4Glcβ1Cer | Merck | GD2 |
| GM1 glycolipid | Galβ1-3GalNAcβ1-4(Neu5Acα2-3)Galβ1-4Glcβ1Cer | Ludger | GM1 |

| | | | |
|---|---|---|---|
| Avanti® is available at Polar Lipids Inc. | | | |

As further standards, the known glycans, subsequent to being labelled with APTS, were digested with at least one of the following exoglycosidases:

| enzyme | supplier | dilution | incubation time |
|---|---|---|---|
| α(2-3,6,8)-Neuraminidase (250 mU/ml) | EY labs | 1:10 | 1 h |
| β(1-3)-Galactosidase (10,000 U/ml) | New England Biolabs® | 1:10 | 3 h |
| β(1-4,6)-Galactosidase (25 U/ml) | Prozyme | 3:10 | over night |
| α(1-3,4.6)-Galactosidase (8,000 U/ml) | New England Biolabs® | 1:10 | 3 h |
| α(1-2,3,4,6)-Fucosidase (2 U/ml) | Prozyme | 4:10 | 24 h |
| β-*N*-Acetylhexosaminidase (5,000 U/ml) | New England Biolabs® | 3:10 | over night |
| β-*N*-Acetylglucosaminidase (4,000 U/ml) | New England | 3:10 | over night |
| α-*N*-Acetylgalactosaminidase (20,000 U/ml) | New England Biolabs® | 1:10 | over night |

Non-specific activity of the exoglycosidases could be excluded by incubation with non-target glycans and subsequent analysis by xCGE-LIF.

The migration times ranging from 15 to 350 MTU measured could be analysed using CorelDRAW 2017. The intensity of the highest peak in each electropherogram was set to 1 for normalizing the relative fluorescence units. For quantitative analysis of peak intensities, the relative signal intensity of each peak was determined. Therefore, the heights of all peaks in the range from 15 to 300 MTU were summed up and the height of individual peaks was calculated as a percentage of the summed-up peak heights. For statistical analysis, the GraphPad Prism 4 software v. 4 was used. In figures, p-values of <0.05 upon unpaired Student's t-test were regarded as statistically significant and are depicted or highlighted by asterisks.

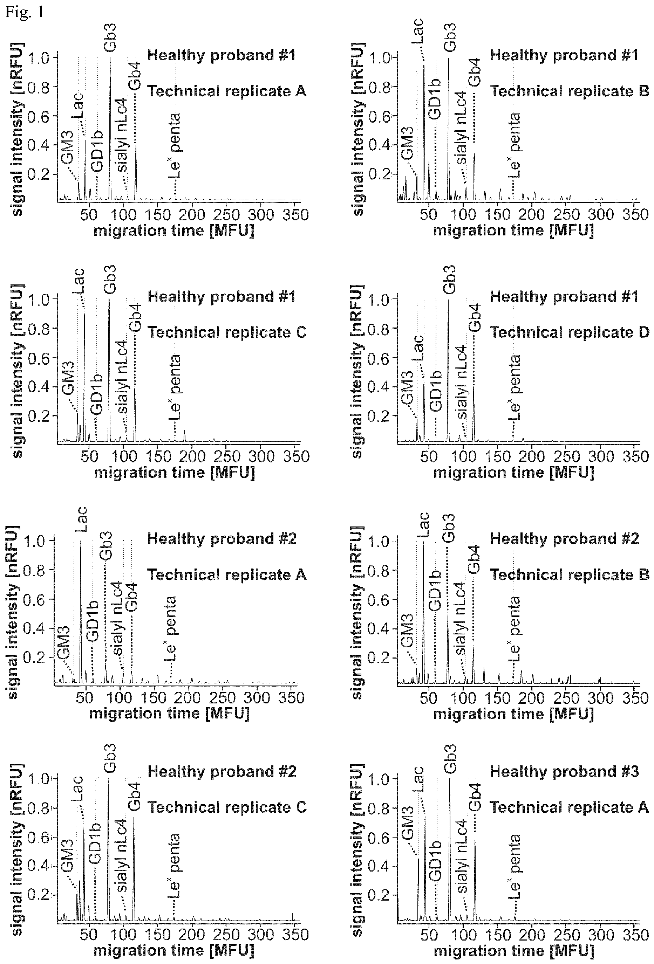

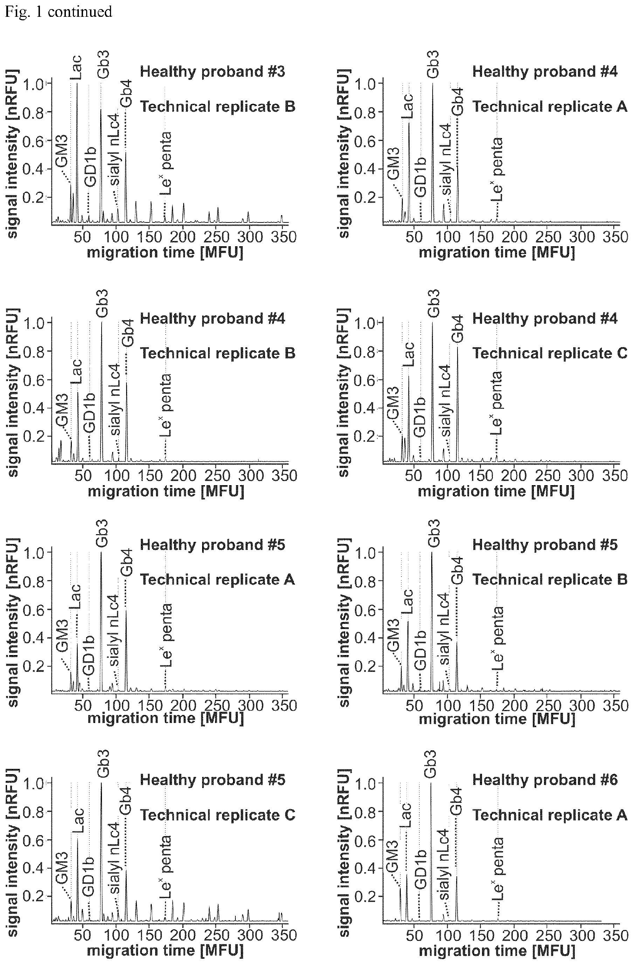

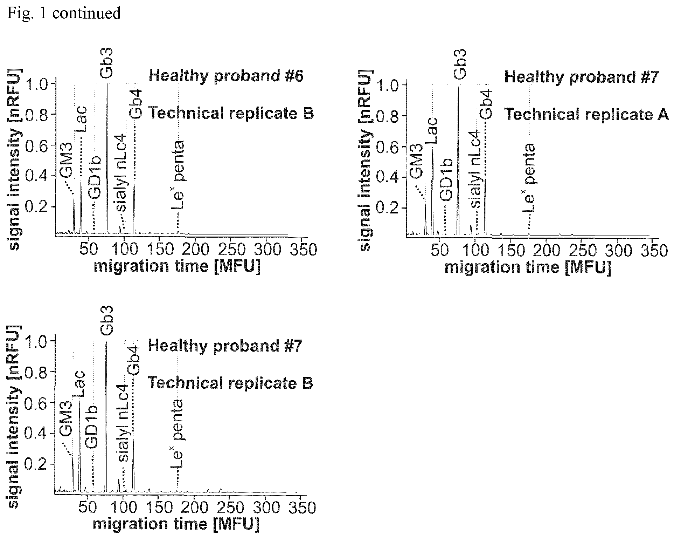

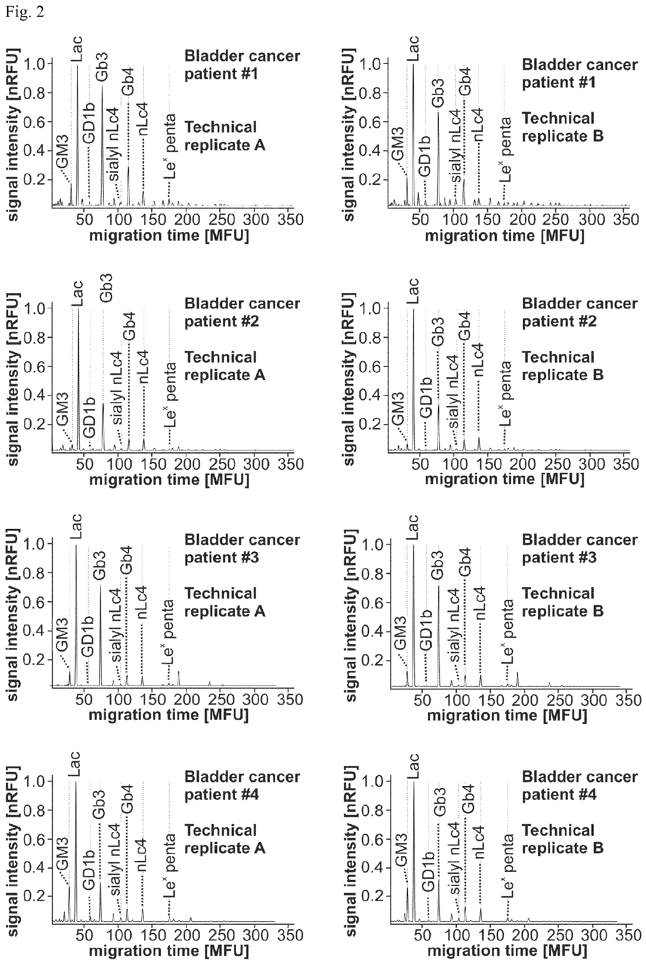

Fig. 1 shows electropherograms obtained by xCGE-LIF of the free glycans, labelled with APTS, obtained by enzymatic hydrolysis of the glycosphingolipids of membrane vesicles from urine samples of healthy persons, and Fig. 2 shows electropherograms of urine samples of diagnosed bladder cancer patients. The glycans indicated were identified using the MTUs of known glycans. The results depicted in Fig. 1 and Fig. 2 show that in urine samples of healthy persons, essentially no nLc4 could be detected in the glycosphingolipid fraction and that in urine samples of bladder cancer patients, nLc4 could be detected in the glycosphingolipid fraction.

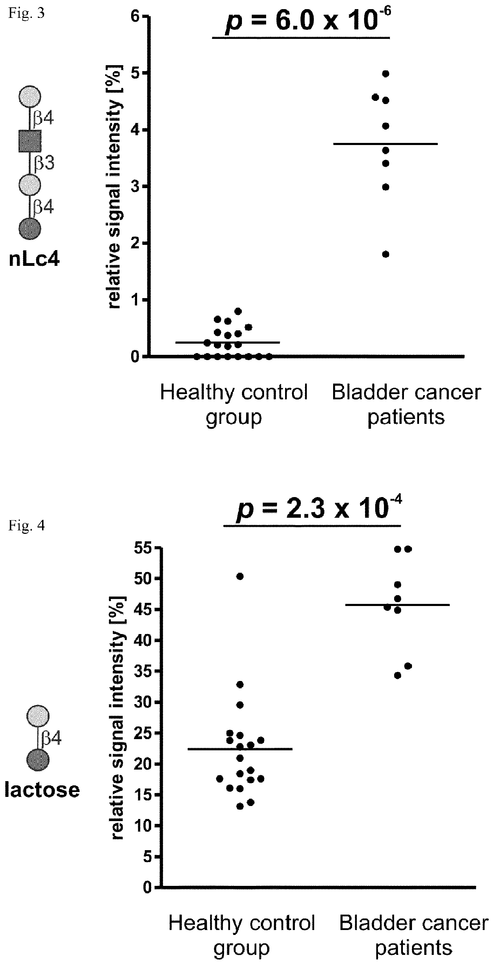

Fig. 3 shows the relative signal intensities for nLc4 of samples from 7 healthy persons with at least two technical replicates, each (left) and from 4 bladder cancer patients with two technical replicates, each (right). As the glycans are labelled at their reducing end with 1 marker molecule, the signal intensity directly corresponds to relative molar concentration. It can be seen that for all healthy samples, the relative signal intensity for nLc4 in relation to the sum of the glycans analysed, which corresponds to the relative molar concentration of the sum of the glycans analysed, is below 1%, with a mean of about 0.2%, whereas for bladder cancer patients, the relative molar concentration is at least 1.0%, with a mean of about 3.7%. The big difference in molar concentration of nLc4 in urine samples of bladder cancer patients shows that nLc4 is a highly specific indicator of bladder cancer, and that the very low or absent concentration of nLc4 in the urine samples of healthy persons avoids false positive indications. Further, the big difference in molar concentration of nLc4 in urine samples of bladder cancer patients allows the detection of nLc4 by a binding peptide having specificity for nLc4, e.g. an antibody specific for nLc4.

Fig. 4 shows the relative signal intensities of lactose in samples from 7 healthy persons with at least two technical replicates, each (left) and from 4 bladder cancer patients with two technical replicates, each (right). It can be seen that the relative signal intensity for lactose, which corresponds to the relative molar concentration, has a mean of about 22% in samples from healthy persons, and a mean of about 46% for samples from bladder cancer patients.

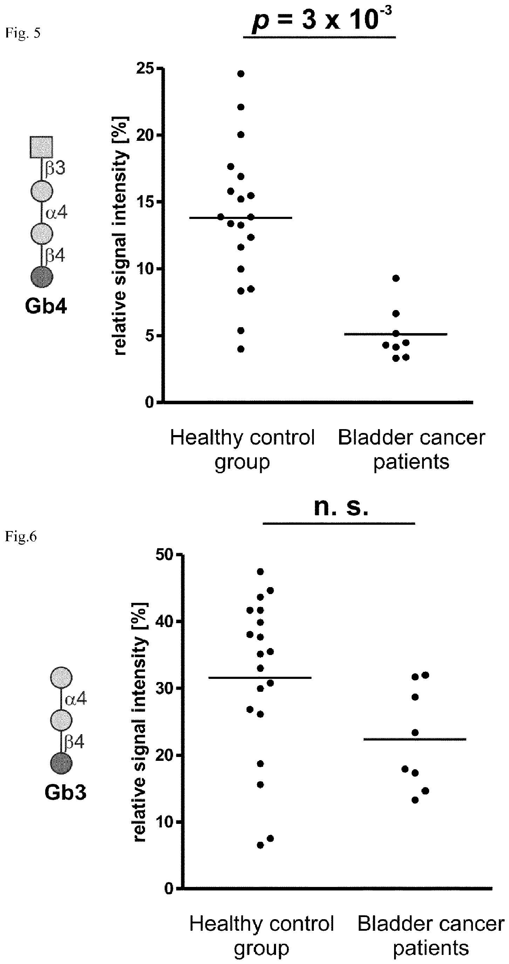

Fig. 5 shows the relative signal intensities of Gb4 of samples from 7 healthy persons with at least two technical replicates, each (left) and from 4 bladder cancer patients with two technical replicates, each (right). It can be seen that the relative signal intensity for Gb4, which corresponds to the relative molar concentration, has a mean of about 14% in samples from healthy persons, and a mean of about 5% for samples from bladder cancer patients.

Fig. 6 shows the relative signal intensities of Gb3 of samples from 7 healthy persons with at least two technical replicates, each (left) and from 4 bladder cancer patients with two technical replicates, each (right). It can be seen that the relative signal intensity for Gb3, which corresponds to the relative molar concentration, has a mean of about 32% in samples from healthy persons, and a mean of about 22% for samples from bladder cancer patients. n.s.: not significant.

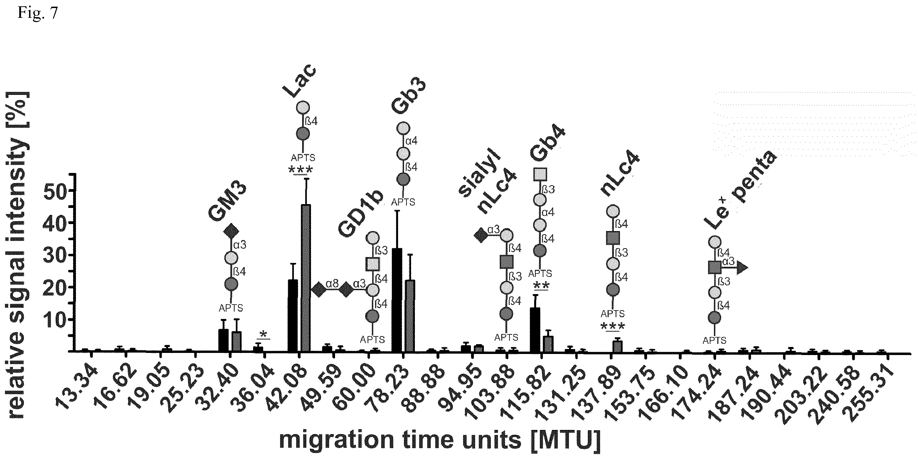

Fig. 7 shows MTUs for some glycans derived from the glycosphingolipids of the membrane vesicle fraction of urine samples of healthy persons (left bar) and of bladder cancer patients (right bar), with the MTU values for the glycans indicated between and below the columns. It becomes clear that an elevated concentration of nLc4 and/or lactose is indicative of bladder cancer and that an elevated concentration of Gb4 and/or Gb3 is indicative of absence of bladder cancer.

### Example 2: Detection of bladder cancer by immunological analysis of nLc4 in urine samples

An antibody against nLc4, e.g. a commercially available antibody or an antibody derived from hybridoma cells generated from animals that were experimentally immunized with nLc4 can be used to detect nLc4 in the urine sample of patients with bladder cancer applying e.g. an ELISA (enzyme linked immuno sorbent assay) or in a RIA (radio immuno assay) or by immunochromatography, e.g. by lateral flow test. The antibody can be polyclonal or monoclonal. The sample could be the glycosphingolipid fraction isolated from a urine sample, with the β-glycosidic bond of glycosphingolipids optionally hydrolysed to release the free glycans. Alternatively, the sample could be the urine sample without pre-treatment, or the sample could be the urine sample, complete or with cells removed, optionally containing an added detergent, further optionally treated with ultrasound, to release glycosphingolipids from membranes. Alternatively or additionally, the urine sample, complete or with cells removed, could be treated by addition of a glycanase, e.g. a ceramide glycanase, to release the glycans from membrane-bound, respectively from solubilized, glycosphingolipids.

### Example 3: nLc4 for use in the immunotherapeutical treatment against bladder cancer.

In this embodiment, immunotherapy includes nLc4 for use in the treatment of bladder cancer. Patients suffering from bladder cancer can be administered (vaccinated) with a glycan comprising or consisting of nLc4 and/or nLc4-ceramide and/or a nLc4-protein conjugate and/or a nLc4-derivative in order to provoke a T-cell dependent or innate immune response against nLc4 in these patients that is then directed against the bladder cancer expressing the antigen epitope nLc4. The immune response can be supported by vaccination in combination with a T-cell carrier (e.g. keyhole limpet hemocyanin) or with adjuvants (e.g. mycobacterium cell-wall skeleton or QS-21 or saponin-based adjuvant).

### Example 4: Immunotherapy against bladder cancer by using an antibody specific for nLc4

In this embodiment, an antibody that is specific for nLc4 is for use in the treatment of bladder cancer, which antibody is directed against nLc4 as a molecular target.

A monoclonal or polyclonal antibody against nLc4, e.g. a commercially available antibody or an antibody derived from hybridoma cells generated from animals that were experimentally immunized with nLc4 can be applied for immunotherapy against bladder cancer. Accordingly, the antibody specific for nLc4 can be a human or a non-human antibody.

For immunotherapy, patients can be directly administered with the nLc4-specific (monoclonal) antibody. This antibody can then activate complement- or cellular-dependent anticancer activities. Such an antibody might be also linked to a cytotoxic agent, e.g. the antibody can be biotinylated and then linked a (strept)avidin-tagged cytotoxic protein, e.g. saporin, a ribosome-inactivating toxin or directly linked to a cytotoxic agent, e.g. ricin chain A toxin or linked to a cytokine. Alternatively an anti-nLc4 antibody can be used to generate a monoclonal anti-idiotype antibody by immunization of experimental animals and generation of hybridoma cells. The anti idiotype monoclonal antibody can be applied as an anti-idiotype vaccine as it mimics the nLc4 epitope and can elicit an anti-nLc4 antibody response that specifically reacts with tumour cells expressing nLc4.

### Example 5: CAR comprising protein binding of nLc4 for use in the treatment of bladder cancer

The paratope of an antibody binding to nLc4 is used to generate a respective single-chain variable fragment (scFv) binding to nLc4. This scFv was used as a portion of a CAR (chimeric antigen receptor), which from N-terminus to C-terminus contained the single-chain variable fragment antibody domain (scFv), a modified hCD8 hinge, a hCD8 transmembrane domain, an intracellular hCD28 signalling domain and an intracellular hCD3ζ (hCD3 zeta) signalling domain, which preferably are linked to one another from N-terminus to C-terminus, more preferably directly linked to one another form N-terminus to C-terminus. The CAR can contain a hΔFc IgG domain in the alternative to a modified hCD8 hinge, and/or a fusion hCD28 transmembrane domain - hCD28/CD3 zeta domain in the alternative to a hCD8 transmembrane domain, an intracellular hCD28 signalling domain and an intracellular hCD3ζ (hCD3 zeta) signalling domain.

A nucleic acid sequence encoding the CAR containing a scFv domain having specificity for nLc4 could be expressed in immune cells, e.g. T-cells, preferably in primary T-cells or NK cells, which cells could be introduced into an experimental animal having bladder cancer. The immune cells expressing the CAR reduced the presence of bladder cancer in the recipient.

## Claims

1. Analytical method for detecting bladder cancer, comprising analysing a urine sample for the presence of the glycan consisting of nLc4.

2. Analytical method according to claim 1, **characterized by** analysing for the presence of the glycan consisting of nLc4 by contacting the sample with a peptide having specificity for binding nLc4.

3. Analytical method according to claim 1, **characterized by** analysing for the presence of the glycan consisting of nLc4 by capillary gel electrophoresis of glycans obtained by hydrolysis of glycosphingolipids of a cell-free membrane vesicle fraction isolated from the urine sample.

4. Analytical method according to one of the preceding claims, **characterized in that** a molar concentration of at least 1 % of nLc4 of the total of glycans originating from glycosphingolipids of the sample indicates presence of bladder cancer.

5. Analytical method according to claim 4, **characterized in that** the molar concentration is determined as the signal intensity.

6. Analytical method according to one of the preceding claims, **characterized by** additionally analysing the urine sample for presence of the glycan consisting of lactose.

7. Analytical method according to one of the preceding claims, **characterized by** additionally analysing the urine sample for presence of the glycan consisting of Gb4.

8. Analytical method according to one of the preceding claims, **characterized by** additionally analysing the urine sample for presence of the glycan consisting of Gb3.

9. Peptide having specificity for binding a glycan consisting of nLc4 for use in the treatment of bladder cancer.

10. Peptide for use in the treatment of bladder cancer according to claim 9, **characterized in that** the peptide is a portion of an antibody or of a CAR.

11. Peptide for use in the treatment of bladder cancer according to one of claims 9 and 10, **characterized in that** the peptide is a CAR expressed in a T-cell.

12. Compound comprising or consisting of nLc4 and/or nLc4-ceramide and/or a nLc4-protein conjugate and/or a nLc4-derivative for use in the treatment of bladder cancer.

13. Compound according to claim 12, wherein the compound is comprised in a formulation suitable for i.m. or for systemic administration.
